# EUROPEAN PATENT APPLICATION

(11) **EP 1 792 647 A1**
(43) Date of publication of application: **06.06.2007**
(21) Application number: 06023678.3
(22) Date of filing: 14.11.2006
(51) Int. Cl.: B01D 47/06, A61L 9/14, F24F 3/16

(54) **Air filtering apparatus**

(30) Priority: 02.12.2005 JP 2005349658
(71) Applicant: SANYO ELECTRIC CO., LTD., Moriguchi-shi Osaka 570-8667 (JP)
(72) Inventor: Usui, Hiroaki, Ora-gun Gunma 370-0533 (JP); Takahashi, Kazuo, Ota-shi Gunma 373-0036 (JP); Suzuki, Daisuke, Ota-shi Gunma 373-0817 (JP); Kurokawa, Keiko, Ora-gun Gunma 370-0517 (JP); Yamamoto, Tetsuya, Ota-shi Gunma 373-0806 (JP); Rakuma, Tsuyoshi, Ora-gun Gunma 370-0524 (JP)
(74) Representative: Glawe, Delfs, Moll

(57) **Abstract**

An air filtering apparatus for subjecting air to sterile filtration to remove microorganisms such as virus, bacteria, etc. from the air and introducing the filtered air to a room including a gas-liquid contact tower (C) having a main body (100) in which electrolytic water and indoor air are supplied to be brought into contact with each other, whereby the indoor air is subjected to sterile filtration with the electrolytic water, the indoor air being introduced from the gas-liquid contact tower into the room.

## Description

### BACKGROUND OF THE INVENTION

### 1. Field of the Invention

The present invention relates to an air filtering apparatus using a gas-liquid contact tower, and particularly to an air filtering apparatus that can remove (containing inactivate, sterilize, etc.) microorganisms such as virus, bacteria, fungus, etc. floating on air.

### 2. Description of the Related Art

There has been proposed a sterilizing apparatus for diffusing electrolytic water mist into air and bringing the electrolytic water mist into direct contact with microorganisms floating on air to inactivate virus, etc. (for example, JP-A-2002-181358).

The above sterilizing apparatus becomes operative under such a use environment that particulate electrolytic water mist easily reach microorganisms, that is, in a relatively small space, however, it hardly becomes operative under such a use environment that the electrolytic water mist hardly reaches microorganisms, that is, in a relatively large space such as a kindergarten, an elementary/junior high/ high school, long-term care insurance facilities, a hospital or the like.

### SUMMARY OF THE INVENTION

The present invention has been implemented in view of the foregoing situation, and has an object to provide an air filtering apparatus that can return filtered air into a room by using a gas-liquid contact tower, whereby the filtered air can be blown out far away in a large space and thus an air filtering efficiency in the large space can be enhanced.

In order to attain the above object, according to an aspect of the present invention, there is provided an air filtering apparatus for subjecting air to sterile filtration to remove microorganisms such as virus, bacteria, etc. from the air and introducing the filtered air to a room, that is characterized by comprising a gas-liquid contact tower (C) having a main body (100) in which electrolytic water and indoor air are supplied to be brought into contact with each other, whereby the indoor air is subjected to sterile filtration with the electrolytic water, the indoor air being introduced from the gas-liquid contact tower into the room.

In the air filtering apparatus, the main body (100) of the gas-liquid contact tower (C) comprises a sprinkler (32) for sprinkling electrolytic water.

In the air filtering apparatus, the main body (100) of the gas-liquid contact tower (C) is equipped with a gas-liquid contact promoting assembly comprising a plurality of plate-shaped gas-liquid contact promoting members (301, 302, 303) arranged in the form of blocks.

In the air filtering apparatus, electrolytic water is supplied from the upper side of the gas-liquid contact promoting assembly into the gas-liquid contact promoting assembly that the electrolytic water falls downwardly along the plate-shaped gas-liquid contact promoting members, and the indoor air is supplied from the lower side of the gas-liquid contact promoting assembly into the gas-liquid contact promoting assembly so that the indoor air is brought into contact with the electrolytic air.

The air filtering apparatus further comprises a supply unit (105) for supplying the electrolytic water to the sprinkler (32), a circulating unit (106) for circulating the electrolytic water sprinkled from the sprinkler (32) to the supply unit (105), and an air intake/exhaust unit (101, 102) for introducing air into the main body of the gas-liquid contact tower and exhausting to the room air that has been subjected to sterile filtration with the electrolytic water in the main body of the gas-liquid contact tower.

In the air filtering apparatus, the supply unit comprises a pump (74) for pumping up the electrolytic water from the main body of the gas-liquid contact tower, and an electrolytic bath (64) in which the water pumped up from the gas-liquid contact tower is electrolyzed to generate the electrolytic water, the electrolytic water generated in the electrolytic bath (64) is circulated to the sprinkler (32).

The air filtering apparatus further comprises a salt solution tank (63) connected to the electrolytic bath (64) to supply salt solution from the salt solution tank to the electrolytic bath.

The air filtering apparatus further comprises a concentration adjusting bath (66) that is disposed between the electrolytic bath (64) and the sprinkler (32) and adjusts the concentration of the salt solution.

The air filtering apparatus further comprises a saturated salt solution tank (61) for supplying salt solution to the salt solution tank (63).

In the air filtering apparatus, the electrolytic bath (64) comprises electrodes (71) for electrolyzing the water to generate the electrolytic water, the polarities of the electrodes being inverted periodically or irregularly under a predetermined condition.

The air filtering apparatus further comprises a mist trap (48) disposed at an indoor-air exit of the main body of the gas-liquid contact tower.

In the air filtering apparatus, the electrolytic water contains active oxygen species generated by electrolyzing tap water, and the active oxygen species contain at least one of hypochlorous acid, ozone and hydrogen peroxide.

The air filtering apparatus further comprises a cooling and heating device comprising an outdoor unit (a) having a heat source side refrigerant cycle and an indoor unit (b) having a use side refrigerant cycle that are connected to each other so that heat exchange can be performed therebetween, wherein the main body of the gas-liquid contact tower is further equipped with an evaporation type heat exchanger (30) that is connected to the use side refrigerant cycle so that at least a part of the refrigerant flowing through the use side refrigerant cycle flows through the evaporation type heat exchanger (30) and heat-exchanges with the electrolytic water supplied to the main body of the gas-liquid contact tower.

According to the present invention, air is subjected to sterile filtration and then returned to a room by using the gas-liquid contact tower. Therefore, the filtered air can be blown out far away in a large space, and the sterile filtration of air in a large space can be efficiently performed.

### BRIEF DESCRIPTION OF THE DRAWINGS

Fig. 1 is a diagram showing the circuit construction of an embodiment of the present invention; and
Fig. 2 is a diagram showing the circuit construction of another embodiment of the present invention.

### DETAILED DESCRIPTION OF THE PREFERRED EMBODIMENTS

Preferred embodiments will be described hereunder with reference to the accompanying drawings.

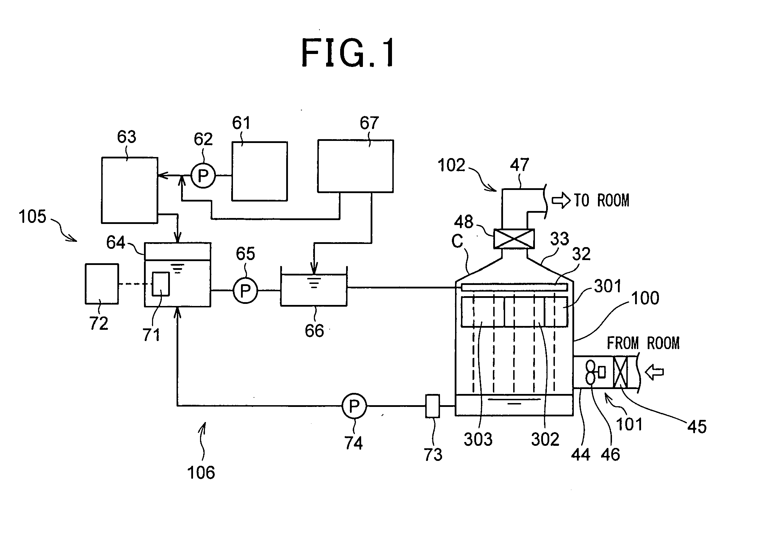

Fig. 1 shows an air filtering apparatus according to an embodiment of the present invention. In this specification, the term "air filtering" is used in a broad sense. For example, "air filtering" means not only removal of contaminants such as microorganisms (virus, bacteria, fungus, etc.) from air, but also inactivation of the microorganisms floating on air even when these microorganisms are not perfectly removed from the air.

This air filtering apparatus has a gas-liquid contact tower c, and the gas-liquid contact tower c has a gas-liquid contact tower body 100. In the gas-liquid contact tower body 100, a plurality of plate-shaped gas-liquid contact promoting members 301, 302, 303, etc. formed of ceramic material are arranged so as to be spaced at an interval in the longitudinal direction while assembled in the form of a block.

A water sprinkler 32 is disposed above the gas-liquid contact promoting members 301, 302, 303, etc. , and the water sprinkler 32 is connected to a supply unit 105 for supplying electrolytic water. The electrolytic water sprinkled from the water sprinkler 32 is passed through the gas-liquid contact promoting members and then circulated to the supply unit 105 by a circulating unit 106. The supply unit 105 comprises a saturated salt solution tank 61, a fist pump 62, a salt solution tank 63, an electrolytic bath 64, a second pump 65, and a concentration adjusting bath 66 which are arranged in this order from the upstream side. Saturated salt solution (or brine) is stocked in the saturated salt solution tank 61, and the saturated salt solution is pumped to the salt solution tank 63 by the first pump 62.

Tap water is appropriately supplied from a tap water stocking tank (or tap water pipe) 67 to the salt solution tank 63, and the concentration of the salt solution in the tank is adjusted to 2 to 3%. This salt solution is supplied to the electrolytic bath 64. A pair of electrodes 71 comprising a cathode and an anode are accommodated in the electrolytic bath 64, and the salt solution is electrolyzed by supplying current to the electrodes 71, thereby generating electrolytic water (0.5 to 1%) containing active oxygen species. Reference numeral 72 represents an electrode controller.

Here, the active oxygen species means oxygen molecules having higher oxidizing activity than normal oxygen and also related substance thereof, and contain not only so-called narrowly-defined active oxygen such as superoxide anion, singlet oxygen, hydroxyl radical and hydrogen peroxide, but also so-called broadly-defined active oxygen such as ozone, hypochlorous acid, etc.

For example when influenza virus invades into indoor air, the active oxygen species function to break down and vanish (remove) the surface protein (spike) of the virus concerned which is indispensable for infection. When the surface protein of influenza virus is broken down, the influenza virus is not joined to a receptor which is necessary for infection of the virus concerned, so that infection can be prevented. As a result of a verification test which was made in cooperation with Sanitary Environment Research, it has been found that when air in which influenza virus invades is brought into contact with electrolytic water, 99% or more of the virus concerned can be removed.

The pair of electrodes 71 are electrode plates each of which includes a base of Ti (titan) and a coating layer of Ir (Iridium), Pt (Platinum), and when the current value applied to the electrodes 71 is set to 20mA(milliampere) /cm² (square centimeter) in current density, a predetermined free residual chlorine concentration (for example, 1mg(milligram)/l(liter) is generated. By supplying current to salt water through the electrodes 71, at the cathode, the following reaction occurs:

4H⁺ + 4e⁻ + (4OH⁻) → 2H₂ + (4OH⁻)

Furthermore, the following reaction occurs at the anode:

2H₂O → 4H⁺ + O₂ + 4e⁻

At the same time, chlorine ions contained water (chlorine ions are added in tap water in advance) react as follows:

2Cl⁻ → Cl₂ + 2e⁻

Furthermore, Cl₂ thus generated reacts with water as follows :

Cl₂ + H₂O → HClO + HCl

The electrolytic water is pumped up by the second pump 65, and fed to the concentration adjusting bath 66. A chlorine sensor (not shown) is disposed in the concentration adjusting bath 66, and water is supplied from the tap water stocking tank 67 into the concentration adjusting bath 66 in accordance with the output of the chlorine sensor. The concentration of chlorine in the bath 66 is adjusted to 10 to 1000ppm, and then the electrolytic water is supplied to the sprinkler 32.

The electrolytic water sprinkled from the sprinkler 32 is brought into contact with the surface of an evaporation type auxiliary heat exchanger 30 (electrolytic water contact portion) while falling downwardly, and then stocked at the lower portion of the gas-liquid contact tower body 100, which is connected to the circulating unit 106 as described above. The circulating unit 106 contains a filter 73 and a third pump 74, and the electrolytic water stocked at the lower portion of the gas-liquid contact tower body 100 is circulated to the electrolytic bath 64 (supply unit) by the third pump 74.

Indoor return air is introduced through an air intake portion 101 into the gas-liquid contact tower body 101, and the air concerned is returned through an air exhaust portion 102 into the room again. The air intake portion 101 contains an air intake duct 44, and a pre-filter 45 and an air blower 46 are disposed in the air intake duct 44. Furthermore, the air exhaust portion (introducing unit) 102 contains the air exhaust duct 47, and a mist trap 48 is disposed in the air exhaust duct 47.

In this construction, by supplying current to the electrodes 71, HCLO (hypochlorous acid) having strong sterilizing power is generated, and it is supplied through the sprinkler 32 into the gas-liquid contact promoting members (electrolytic water contact portions) 301, 302, 303, etc. The electrolytic water from the sprinkler 32 falls downwardly along the plates (gas-liquid contact promoting members) from the upper side to the lower side between the gas-liquid contact promoting members 301, 302, 303, etc., and also the indoor return air is blown by the air blower 46 and circulated from the lower side to the upper side along the plates.

Accordingly, virus floating in air passing through the gas-liquid contact promoting members can be inactivated by the electrolytic water, and also various kinds of virus, bacteria, etc. can be prevented from breeding on the surface of the gas-liquid contact members.

Furthermore, odor components also react with hypochlorous acid in the electrolytic water when passing there, so that the odor components are ionized, dissolved and thus removed from the air. Therefore, the air is deodorized. Here, the filtered (inactivated, sterilized or the like) and deodorized air is blown by the air blower 46 and returned through the air exhaust duct 47 to a large space in which many persons gather together, which is called as an arena, a dome or the like.

In this construction, filtered air is returned to the room by using the cleaning tower type gas-liquid contact tower c, and thus the filtered air can be blown out far away in a large space, and thus the air filtering efficiency in the large space can be efficiently performed.

In the gas-liquid contact tower c, the indoor return air taken by the air blower 46 and the water dropped from the sprinkler 32 disposed at the upper side are brought into contact with each other. Therefore, the air taken into the gas-liquid contact tower contains water vapor generated through vaporization of a part of the water, and reaches as high-temperature and high-humidity air to the air exhaust portion 102.

When this air is left as it is, the high-temperature and high-humidity air is condensed in the process that it is cooled in atmospheric air, and a lot of fog and steam occur. In this construction, the mist trap 48 is disposed in the air exhaust duct 47, and thus the mist component in the air is removed. Accordingly, the condensation of the air is stopped in the progress of cooling, and occurrence of steam is suppressed.

The gas-liquid contact promotingmembers 301, 302, 303, etc. are formed of ceramic material so as to prevent adherence of scale to them.

Even when scale adheres to the gas-liquid contact promoting members, the gas-liquid contact promoting member can be exchanged every block because the plural plate-shaped gas-liquid contact promoting members 301, 302, 303, etc. are arranged to be assembled in the form of blocks, or scale can be removed after each block is taken out. Therefore, the maintenance can be easily performed.

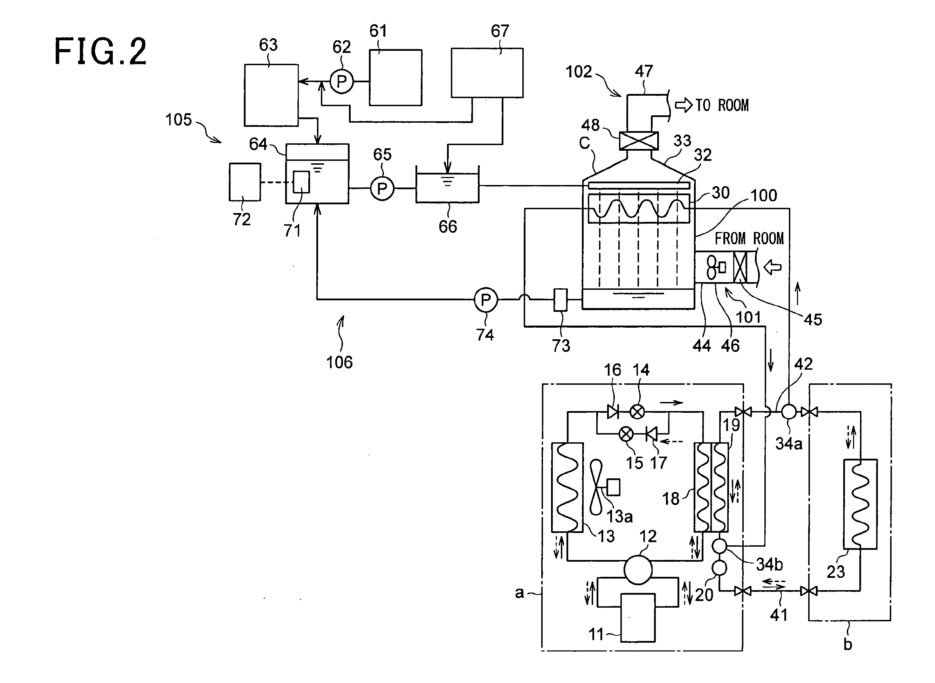

Fig. 2 shows another embodiment of the air filtering apparatus of the present invention.

In this embodiment, the gas-liquid contact tower c is constructed in combination with a cooling and heating device. The cooling and heating device comprises an outdoor unit a, an indoor unit b and a gas-liquid contact tower (cleaning tower unit)c.

Reference numeral 11 represents a compressor, reference numeral 12 represents a four-way valve, reference numeral 13 represents a heat-source side heat exchanger, reference numeral 13a represents an air blower, reference numeral 14 represents a pressure reducing device for cooling, reference numeral 15 represents a pressure reducing device for heating, reference numeral 16 represents a check valve for closing the pressure reducing device 14 for cooling during heating operation, reference numeral 17 represents a check valve for closing the pressure reducing device 15 for heating during cooling operation, and reference numeral 18 represents a first auxiliary heat exchanger. These elements are connected to one another in a ring shape to form a heat-source side refrigerant cycle. Reference numeral 19 represents a second auxiliary heat exchanger, and it is integrally formed with the first auxiliary heat exchanger 18 to carry out the heat exchange with the first auxiliary heat exchanger 18. Reference numeral 20 has a refrigerant feeding device, and has the reversible characteristic that the flow-out direction of refrigerant is opposite between the cooling operation and the heating operation. These elements are accommodated in the outdoor unit a.

A use-side heat exchanger 23 is accommodated in the indoor unit b, and the second auxiliary heat exchanger 19 is annularly connected to the use-side heat exchanger 23 through connection pipes 41, 42, thereby forming a user-side refrigerant cycle.

An evaporation type auxiliary heat exchanger 30 for radiating heat of the use-side refrigerant cycle is disposed in the gas-liquid contact tower body 100 of the gas-liquid contact tower c.

The evaporation type auxiliary heat exchanger 30 comprises plural plate type heat exchangers, and they are disposed in the blocked arrangement, for example. Accordingly, these plate type heat exchangers can be exchanged or reproduced every block. The evaporation type auxiliary heat exchanger 30 and the second auxiliary heat exchanger 19 are provided in series, and cools the refrigerant by evaporation latent heat of water. This auxiliary heat exchanger 30 serves as an electrolytic water contact portion as described later. Reference numerals 34a, 34b represent three-way flow amount valves for adjusting the flow of the refrigerant of the use-side refrigerant cycle to the evaporation type auxiliary heat exchanger 30.

Indoor return air is introduced through the air intake portion 1010 into the gas-liquid contact tower body 100, and the air concerned is returned through the air exhaust portion 102 into the room. The air intake portion 101 contains the air intake duct 44, and the pre-filter 45 and the air blower 46 are disposed in the air intake duct 44. Furthermore, the air exhaust portion (introducing unit) 102 contains the air exhaust duct 47, and the mist-trap 48 is disposed in the exhaust duct 47.

In the gas-liquid contact tower c, the indoor return air taken by the air blower 46 and the water dropped from the sprinkler 32 at the upper side are brought into contact with each other, and the evaporation type auxiliary heat exchanger 30 is cooled by using the latent heat when the water is evaporated. Therefore, the air taken into the gas-liquid contact tower contains water vapor generated through evaporation of a part of the water, and thus it reaches the air exhaust portion 102 as high-temperature and high-humidity air.

When this air is left as it is, the high-temperature and high-humidity air is condensed in the process that it is cooled in atmospheric air, and a lot of fog and steam occur. In this construction, the mist trap 48 is disposed in the air exhaust duct 47, and thus the mist component in the air is removed. Accordingly, the condensation of the air is stopped in the progress of cooling, and occurrence of steam is suppressed.

The supply unit 105 supplying electrolytic water to the sprinkler 32 and the circulating unit 106 for circulating the electrolytic water to the supply unit 105 have the same constructions as the above-described embodiment. Therefore, the same parts are represented by the same reference numerals and the description thereof is omitted.

In this construction, by supplying current to the electrodes 71, HC10 (hypochlorous acid) having strong sterilizing power is generated, and hypochlorous acid is supplied to the surface of the evaporation type auxiliary heat exchanger 30 (electrolytic water contact portion). Accordingly, various kinds of bacteria, etc. canbe prevented from breeding on the surface of the evaporation type auxiliary heat exchanger 30, and also virus, etc. floating in the air passing there can be inactivated. Furthermore, odor reacts with hypochlorous acid in the electrolytic water when passing through the evaporation type auxiliary heat exchanger 30, and it is ionized and dissolved, whereby the odor is removed from the air and thus the air is deodorized.

The electrolytic water sprinkled from the sprinkler 32 is brought into contact with the surface of the evaporation type auxiliary heat exchanger 30, falls downwardly and then is stocked at the lower portion of the gas-liquid contact tower body 100. The circulating unit 106 is connected to the lower portion concerned.

Next, the operation of the cooling and heating device will be described.

In the normal cooling operation when the outside air temperature is high, the refrigerant cycle indicated by a solid line in Fig. 2 is carried out. In the heat-source side refrigerant cycle, high-temperature and high-pressure gas refrigerant from the compressor 11 is passed through the four-way valve 12, and radiates heat to be condensed and liquefied in the heat-source side heat exchanger 13. Then, the condensed and liquefied refrigerant is passed through the check valve 16, and reduced in pressure in the cooling expansion valve 14. Furthermore, the refrigerant is evaporated in the first auxiliaryheat exchanger 18, passed through the four-way valve 12, and then circulated to the compressor 11.

At this time, the second auxiliary heat exchanger 19 and the firsts auxiliary heat exchanger 18 of the use-side refrigerant cycle are heat-exchanged with each other to cool and liquefy the gas refrigerant in the use-side refrigerant cycle. Then, the liquefied refrigerant is fed to the refrigerant feeding device 20, and it is passed through the connection pipe, and fed to the use-side heat exchanger 23 to be cooled and endothermically evaporated. The gasified refrigerant is passed through the connection pipe and circulated to the second auxiliary heat exchanger 19.

In the cooling operation when the outside air temperature is low (for example, 0°C), the opening degree of the three-way flow amount valves 34a, 34b is adjusted as occasion demands in addition to the operation of the use-side refrigerant cycle, and the refrigerant flow to the evaporation type auxiliary heat exchanger 30 is controlled.

At the same time, the first, second, third pumps 62, 65 and 74 are driven, the supply unit 105 and the circulating unit 106 are driven so that the electrolytic water is circulatingly sprinkled to the evaporation type auxiliary heat exchanger 30 through the sprinkler 32 to cool the evaporation type auxiliary heat exchanger 30 with the evaporation latent heat of the electrolytic water. The cooled and liquefied refrigerant flows into the use-side heat exchanger 23 by the refrigerant feeding device 20, thereby forming the cooling cycle.

At this time, when cooling capacity (power) is insufficient, the heat-source side refrigerant cycle is driven with proper capacity (power) so that the refrigerant adjusted by the three-way flow amount valve 34a is cooled in the second auxiliary heat exchanger 19, is made confluent with the refrigerant from the use-side heat exchanger 23 in the three-way flow amount valve 34b, and then fed to the refrigerant feed device 20 to thereby perform cooling operation.

Under heating operation, the refrigerant cycle indicate by a broken line in Fig. 2 is established. In the heat-source side refrigerant cycle, the high-temperature and high-pressure refrigerant from the compressor 11 is fed from the four-way valve 12 to the first auxiliary heat exchanger 18 to radiate heat, so that it is condensed and liquefied. The liquefied refrigerant is passed through the check valve 17 to the heating pressure-reducing device to be reduced in pressure. Furthermore, the pressure-reduced refrigerant is endothermically evaporated in the heat source side heat exchanger 13, passed through the four-way valve 12 and then circulated to the compressor 11. At this time, the second auxiliary heat exchanger of the use-side refrigerant cycle and the first auxiliary heat exchanger 18 carry out the heat exchanger therebetween. The liquid refrigerant in the use-side refrigerant cycle is heated and gasified, and fed to the use-side heat exchanger 23. Then, the refrigerant is heated and liquefied, fed to the refrigerant feeding device 20 and circulated to the second auxiliary heat exchanger 19.

The above embodiment is an example of the cooling and heating device, and thus the cooling and heating device is not limited to the above embodiment, and any construction may be adopted for the cooling and heating device.

In this embodiment, the electrolytic water is circulatingly sprinkled to the evaporation type heat exchanger 30 of the gas-liquid contact tower by the mechanism for supplying the electrolytic water, and the indoor return air is circulated to the evaporation type auxiliary heat exchanger 30 to filter the indoor return air with the electrolytic water. The indoor return air thus filtered is returned to the room. Therefore, even in a kindergarten, an elementary/junior high/ high school, long-term care insurance facilities, a hospital or the like or in a large space in which many persons gather, for example, so-called arena, dome or the like, filtered (sterilized, inactivated or the like) air can be blown out far away and the sterile filtration of air in a large space can be efficiently performed.

The present invention is not limited to the above embodiment. For example, the air filtering apparatus of the present invention may be designed so that ozone (O₃) or hydrogen peroxide (H₂O₂) is generated as the active oxygen species. In this case, when platinum tantalum electrodes are used as the electrodes, active oxygen species can be highly efficiently and stably generated from water in which ion species are rare.

At this time, at the anode, the following reaction occurs:

2H₂O → 4H⁺ + O₂ + 4e⁻

Simultaneously with the above reaction, the following reactions occur, and ozone (O₃) is generated.

3H₂O → O₃ + 6H⁺ + 6e⁻

2H₂O → O₃ + 4H⁺ + 4e⁻

Furthermore, at the cathode, the following reactions occur:

4H⁺ + 4e⁻ + (4OH⁻) → 2H₂ + (4OH⁻)

O₂⁻ + e⁻ + 2H⁺ → H₂O₂

That is, O₂⁻ generated through the electrode reaction and H⁺ in solution are bonded to each other to generate hydrogen peroxide (H₂O₂).

In this construction, ozone (O₃) and hydrogen peroxide (H₂O₂) which have strong sterilizing power are generated by supplying current to the electrodes, and electrolytic water containing ozone (O₃) and hydrogen peroxide (H₂O₂) can be created. The concentration of ozone or hydrogen peroxide in the electrolytic water is adjusted to a value suitable for inactivate target virus or the like and air is passed through the gas-liquid contact member 5 supplied with the electrolytic water having this concentration, whereby target virus, etc. floating in the air can be inactivated. Furthermore, even odor reacts with ozone or hydrogen peroxide in the electrolytic water when passing through the gas-liquid contact member 5, and ionized and dissolved in the electrolytic water, whereby the odor components are removed from the air and thus the air is deodorized.

When scale deposits on the electrode (cathode) by electrolyzing tap water, the electrical conductivity is lowered, and it is difficult to continue electrolysis.

In this case, it is effective to invert the polarities of the electrodes (the plus and minus of the electrodes are switched to each other). The scale depositing on the cathode can be removed by electrolyzing the cathode as the anode. With respect to this polarity inverting control, the inverting operation may be periodically carried out by using a timer, for example, or it may be carried out irregularly, for example, every time the operation is started. Furthermore, the increase of the electrolysis resistance (decrease of the electrolysis current, or increase of the electrolysis voltage) is detected, and the polarities may be inverted on the basis of the detection result.

In the above embodiments, the water supply system using the tap water stock tank 67 is used. However, in place of the tap water stock tank 67, the apparatus may be connected to a tap water pipe, that is, a water-pipe based water supply system for directly introducing city water may be used.

Furthermore, the above construction of the gas-liquid contact mechanism is not limited to the cooling tower, but it may be applied to a so-called heating tower or the like which uses brine contaminated with antifreeze solution.

In the above embodiment, tap water (city water) is used as the water from which electrolytic water is generate is tap water. However, the water being used is not limited to the tap water, and any kind of water may be used insofar as active oxygen species can be generated from the water.

In the above embodiment, indoor air in a room is passed through the air filtering apparatus to be subjected to sterile filtration, and then the filtered indoor air is returned to the room. However, outside air may be first passed through the air filtering apparatus to be subjected to sterile filtration, and then introduced into the room.

## Claims

1. An air filtering apparatus for subjecting air to sterile filtration to remove microorganisms such as virus, bacteria, etc. from the air and introducing the filtered air to a room, **characterized by** comprising a gas-liquid contact tower (C) having a main body (100) in which electrolytic water and indoor air are supplied to be brought into contact with each other, whereby the indoor air is subjected to sterile filtration with the electrolytic water, the indoor air being introduced from the gas-liquid contact tower into the room.

2. The air filtering apparatus according to claim 1, wherein the main body (100) of the gas-liquid contact tower (C) comprises a sprinkler (32) for sprinkling electrolytic water.

3. The air filtering apparatus according to claim 1, wherein the main body (100) of the gas-liquid contact tower (C) is equipped with a gas-liquid contact promoting assembly comprising a plurality of plate-shaped gas-liquid contact promoting members (301, 302, 303) arranged in the form of blocks.

4. The air filtering apparatus according to claim 3, wherein the electrolytic water is supplied from the upper side of the gas-liquid contact promoting assembly into the gas-liquid contact promoting assembly that the electrolytic water falls downwardly along the plate-shaped gas-liquid contact promoting members, and the indoor air is supplied from the lower side of the gas-liquid contact promoting assembly into the gas-liquid contact promoting assembly so that the indoor air is brought into contact with the electrolytic air.

5. The air filtering apparatus according to claim 2, further comprising a supply unit (105) for supplying the electrolytic water to the sprinkler (32), a circulating unit (106) for circulating the electrolytic water sprinkled from the sprinkler (32) to the supply unit (105), and an air intake/exhaust unit (101, 102) for introducing air into the main body of the gas-liquid contact tower and exhausting to the room air that has been subjected to sterile filtration with the electrolytic water in the main body of the gas-liquid contact tower.

6. The air filtering apparatus according to claim 5, wherein the supply unit comprises a pump (74) for pumping up the electrolytic water from the main body of the gas-liquid contact tower, and an electrolytic bath (64) in which the water pumped up from the gas-liquid contact tower is electrolyzed to generate the electrolytic water, the electrolytic water generated in the electrolytic bath (64) is circulated to the sprinkler (32).

7. The air filtering apparatus according to claim 6, further comprising a salt solution tank (63) connected to the electrolytic bath (64) to supply salt solution from the salt solution tank to the electrolytic bath.

8. The air filtering apparatus according to claim 6, further comprising a concentration adjusting bath (66) that is disposedbetween the electrolytic bath (64) and the sprinkler (32) and adjusts the concentration of the salt solution.

9. The air filtering apparatus according to claim 7, further comprising a saturated salt solution tank (61) for supplying salt solution to the salt solution tank (63).

10. The air filtering apparatus according to claim 6, wherein the electrolytic bath (64) comprises electrodes (71) for electrolyzing the water to generate the electrolytic water, the polarities of the electrodes being inverted periodically or irregularly under a predetermined condition.

11. The air filtering apparatus according to claim 1, further comprising a mist trap (48) disposed at an indoor-air exit of the main body of the gas-liquid contact tower.

12. The air filtering apparatus according to claim 1, wherein the electrolytic water contains active oxygen species generated by electrolyzing tap water, and the active oxygen species contain at least one of hypochlorous acid, ozone and hydrogen peroxide.

13. The air filtering apparatus according to claim 1, further comprising a cooling and heating device comprising an outdoor unit (a) having a heat source side refrigerant cycle and an indoor unit (b) having a use side refrigerant cycle that are connected to each other so that heat exchange can be performed therebetween, wherein the main body of the gas-liquid contact tower is further equipped with an evaporation type heat exchanger (30) that is connected to the use side refrigerant cycle so that at least a part of the refrigerant flowing through the use side refrigerant cycle flows through the evaporation type heat exchanger (30) and heat-exchanges with the electrolytic water supplied to the main body of the gas-liquid contact tower.
